# EUROPEAN PATENT SPECIFICATION

(11) **EP 3 138 600 B1**
(45) Date of publication and mention of the grant of the patent: **06.11.2019**
(21) Application number: 15183956.0
(22) Date of filing: 04.09.2015
(51) Int. Cl.: A61M 15/00

(54) **DRY POWDER INHALER**
TROCKENPULVERINHALATOR
INHALATEUR DE POUDRE SÈCHE

(43) Date of publication of application: 08.03.2017
(73) Proprietor: Esteve, Victor, 13.306-530 Itu (BR)
(72) Inventor: Esteve, Victor, 13.306-530 Itu (BR)
(74) Representative: DREISS Patentanwälte PartG mbB

(56) References cited:
- EP-A2- 1 270 034
- WO-A1-2015/006838
- US-A- 3 858 583
- US-A- 5 797 391
- US-A1- 2015 231 344

## Description

The invention relates to a dry powder inhaler for a capsule containing dry powder, the inhaler comprising a base body having a capsule receptacle, two actuator buttons arranged on opposing sides of the base body and two perforation needles, each needle being fixedly connected to an actuator button and movable relative to the base body towards each other from a normal position to a perforation position along an actuation direction to perforate a capsule arranged in the capsule receptacle.

Such a dry powder inhaler is known, for example from EP 1 270 034 A1 or from US 2003/0000523. Those dry powder inhalers are typically non-pressurized inhalators. A capsule which is arranged in the capsule receptacle of the dry powder inhalator can be pierced by the needles. When air is sucked in by a patient through a mouthpiece, the capsule is lifted from the capsule receptacle into a capsule chamber and rotates in the capsule chamber. Upon rotation of the capsule, dry powder contained in the capsule is dispersed by centrifugal forces during rotation and mixed with air such that it can be inhaled by a patient.

A problem of the known dry powder inhalers is that the dosage of dry powder is limited. When piercing the capsule directly in its hemispherical ends, the amount of powder to be dispersed during inhalation is limited because the flow of the dry powder out of the pierced openings is obstructed during rotational movement of the capsule due to the arrangement of the openings. The obstruction can be caused by cut-out portions of the capsule shell which are cut out by the needles when piercing the capsule. Moreover, the known dry powder inhalers are limited in the maximum dosage of dry powder. In certain areas of application a high dosage in the range of about 25 to about 200mg of dry powder is required such as treatments or clinical tests for cystic fibrosis, tuberculosis, pulmonary artery hypertension, neurologic and congenital disorders, Parkinson, asthma, Chronic obstructive pulmonary disease (COPD) etc. Another dry powder inhaler is for example known from WO 2015/006838 A1. Other examples of inhales are disclosed in US 2015/231344A1, US 3858583A and US 5797391A.

It is therefore an object of the present invention to provide a cost effective dry powder inhaler for dispersion of dry powder which provides for easy handling of large dosages of dry powder.

This object is solved by a dry powder inhalator according to the features of claim 1. Such a dry powder inhalator is characterized in that the capsule receptacle is arranged in an inclined angle within the range of about 40° to about 50° with respect to the actuation direction. Arranging the capsule receptacle in such an inclined manner with respect to the actuation direction provides the advantage that a capsule which can be arranged in the capsule receptacle can be pierced in a transition area of the hemispherical ends and the tubular middle section of the capsule. Preferably, the capsule receptacle is arranged such that a capsule can be pierced in a position that allows powder to be dispersed during the rotational movement in an inhalation process while providing little or almost no obstruction to the powder flow.

According to a first particularly preferred embodiment of the dry powder inhaler, the needles are arranged parallel to the actuation direction and spaced apart from a middle plane of the base body. The middle plane is a plane parallel to a longitudinal axis of the base body which divides the base body into two parts of substantially the same size. By arranging the needles parallel to the actuation direction and spaced apart from the middle plane of the base body, a capsule arranged in the capsule receptacle can be pierced in an angle such that a cut-out portion of the capsule can be bent into the capsule such that it does not obstruct the flow of dry powder during inhalation. Preferably, the needles are arranged on opposing sides of the middle plane. Therefore, upon piercing a capsule arranged in the capsule receptacle, the cut-out portions can be bent inside into the capsule in a flap-like manner.

According to another preferred embodiment, the capsule receptacle is arranged perpendicular to a longitudinal axis of the inhaler.

It is particularly preferred it the base body comprises two hollow needle guiding sections configured to guide the needles, the needle guiding sections being arranged perpendicular to a longitudinal axis of the inhaler and ending in the capsule receptacle. By providing such needle guiding sections the needles can be guided and supported during actuation of the actuator buttons such that a capsule inserted into the capsule receptacle is always pierced in substantially the same position.

According to another advantageous embodiment, the base body is having a capsule chamber for rotation of a capsule. Providing such a capsule chamber has the advantage that a capsule can be lifted into the capsule chamber by sucking air through a mouthpiece of the dry powder inhalator and rotate in the capsule chamber due to air flow generated in the capsule chamber upon inhalation.

Therefore, it is particularly preferred if the capsule receptacle is arranged in the capsule chamber and connected to the capsule chamber.

Preferably, the capsule chamber is having a circular cylindrical section and a conical section, wherein the capsule receptacle is arranged in the conical section.

In a preferred embodiment, the circular cylindrical section of the capsule chamber has diameter which is bigger than a length of the capsule receptacle. The diameter is preferably chosen such that a capsule can be lifted into the capsule chamber during inhalation and rotates in the capsule chamber.

In order to provide for a rotational movement of the capsule during inhalation it is particularly preferred if the base body is having at least two air inlets connected to the capsule chamber and ending tangentially in the capsule chamber. Advantageously the air inlets end into the capsules chamber perpendicular to a middle plane of the base body. Preferably, the air inlets end in the circular cylindrical section of the capsule chamber. When air is sucked in through a mouthpiece of the dry powder inhalator which is in fluid communication with the capsule chamber, ambient air can be sucked into the capsule chamber through the air inlets such that a capsule is lifted and rotates in the capsule chamber.

In order to conduct ambient air to the air inlets of the base body it is particularly preferred if the dry powder inhaler comprises a mouthpiece arranged on top of the base body, wherein the mouthpiece comprises at least one, advantageously two air inlets, fluidly connected with the air inlets of the base body. Preferably, those air inlets of the mouthpiece are arranged on a lateral side of the mouthpiece. Advantageously, the inhaler body functions as an air buffer area wherein air is sucked into the capsule chamber during inhalation through the air inlets of the base body via the air inlets of the mouthpiece.

According to a preferred embodiment, the capsule receptacle is configured to receive a capsule with a volume in the range of about 400 to about 500 mm³. Advantageously, such a capsule has a volume of 437 mm³. Preferably, the size of the capsule receptacle corresponds with the size of the capsule such that the capsule receptacle is only slightly bigger than the capsule to ensure that the capsule is held in the capsule receptacle when pushing the actuator buttons and piercing the capsule with the needles.

If is particularly preferred if the capsule chamber has a volume of about 2500 to about 3000 mm³. By providing a capsule chamber with such a volume it can be ensured that the capsule can rotate efficiently during inhalation.

Advantageously, the needles have a needle diameter of about 1,55 to about 1,85 mm. With such a needle diameter, the release of high dosage of dry powder can be insured.

According to another preferred embodiment of the dry powder inhaler, the needles have a cutting tip with a cutting tip angle with respect to a longitudinal axis of the needle in the range of about 20 to about 35°. Such a cutting tip angle can allow an accurate cutting of a shell of a capsule during piercing of the capsule without detaching the cut-out portion from the capsule shell. Therefore, the cut-out portion can be bent inwards in a hinged fashion.

It is particularly preferred if the needles have lateral cutting edges which are arranged in an angle in the range of about 25 to 35° with respect to an axis perpendicular to the longitudinal axis and lying in a plane parallel to the longitudinal axis.

Advantageously, the needles are symmetrical to a middle plane.

Further details and advantages of the invention can be taken from the following description, on the basis of which the embodiments of the invention that are represented in the Figures are described and explained in more detail.

Respective figures are showing:
- Figure 1: a side view of a dry powder inhalator according to the invention;
- Figure 2: a perspective view of the dry powder inhalator of Figure 1;
- Figure 3: a perspective view showing parts of the dry powder inhalator of Figures 1 and 2;
- Figure 4: a perspective view of a base body of the dry powder inhalator of Figures 1 to 3;
- Figure 5: a perspective top view of the base body of Figure 4;
- Figure 6: a top view of the base body of Figures 4 and 5;
- Figure 7: a capsule and two needles in a piercing position;
- Figure 8: a side view of a needle of the dry powder inhalator of Figures 1 to 4:
- Figure 9: a top view of the needle of Figure 8;
- Figure 10: a front view of the needle of Figures 8 and 9; and;
- Figure 11: schematic view of a pierced capsule during inhalation.

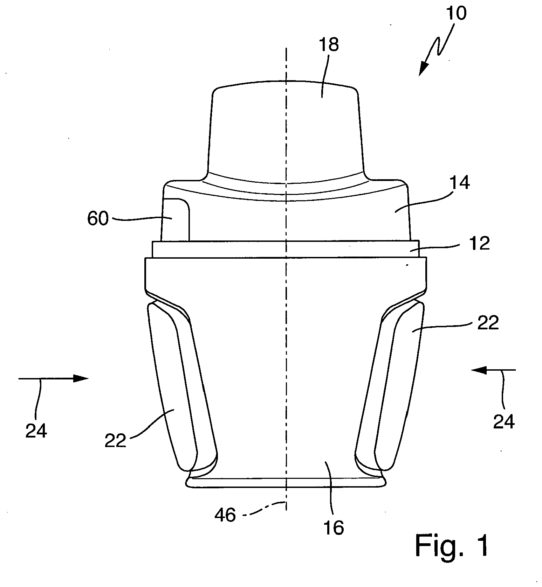

Figures 1 to 10 show a dry powder inhaler 10 for a capsule containing dry powder (not shown in the drawings). The dry powder inhaler 10 comprises a base body 12, a mouthpiece 14 and a covering body 16. The mouthpiece 14 has a mouth portion 18 having a central opening 20 for inhaling air that mixes with dry powder contained in a capsule arranged in the dry powder inhaler 10.

The dry powder inhaler 10 comprises two actuator buttons 22 arranged on opposing sides of the base body 12 and are movable relative to the base body 12 along an actuation direction which is indicated by arrows 24 from a normal position to a perforation position to perforate a capsule arranged in the dry powder inhaler 10.

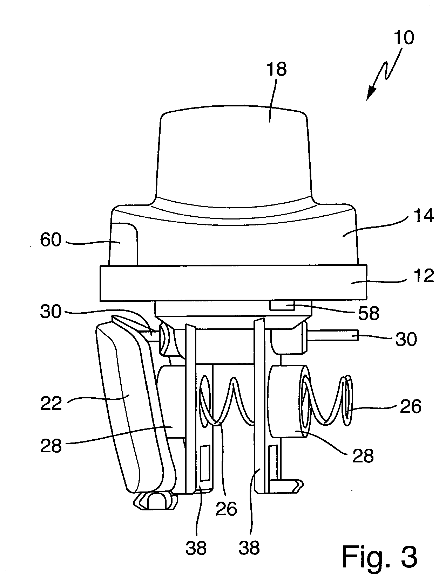

Figure 3 shows a perspective view with of parts of the dry powder inhaler 10 to facilitate the description of the operation of the dry powder inhaler 10. The dry powder inhaler 10 comprises a helical spring 26 arranged in shell-like spring-guiding members 28 of the base body 12 and acting upon the actuator buttons 22 such that the actuator buttons 22 are pre-loaded into the normal position against the direction depicted by arrows 24.

The dry powder inhaler 10 furthermore comprises two perforation needles 30, wherein each perforation needle 30 is fixedly connected to an actuator button 22.

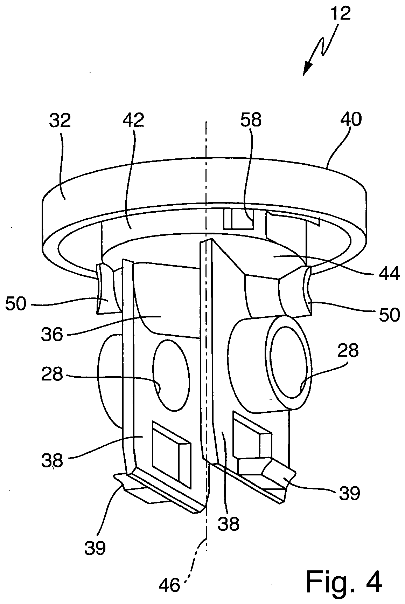

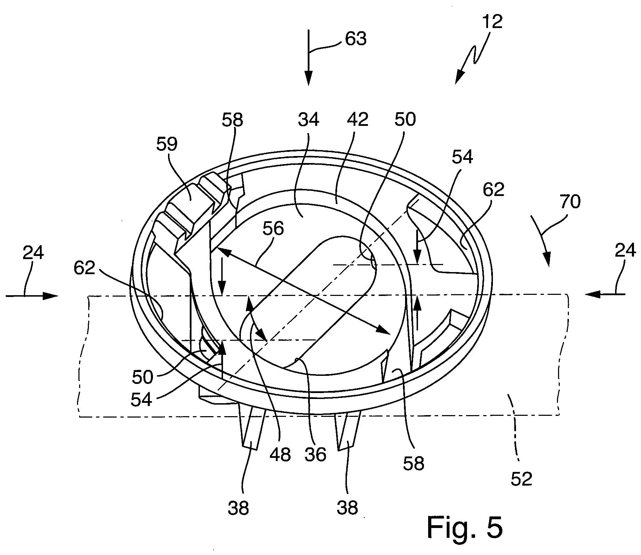

The base body 12 of the dry powder inhaler 10 which is made of one piece is shown in more detail in Figures 4 and 5. The base body has an elliptical collar section 32, a capsule chamber 34 having a capsule receptacle 36 for receiving a dry powder capsule and two downwardly extending plate members 38 that are substantially parallel to each other. The spring-guiding members 28 are arranged coaxially to the actuation direction 24 wherein the plate members 38 are arranged perpendicular to the actuation direction 24. At a free end of the plate members 38, bearing sections 39 for the actuator buttons 22 are provided .

The capsule chamber 34 is open towards an upper end portion 40 of the base body 12. The capsule chamber 34 has a circular cylindrical section 42 and a conical section 44, wherein the capsule receptacle 36 is arranged in the conical section 44 of the capsule chamber 34. The capsule chamber 34 has a volume of about 2500 to about 3000 mm³. The capsule receptacle 36 is arranged perpendicular to a longitudinal axis 46 of the dry powder inhaler 10.

The capsule receptacle 36 is furthermore arranged in an inclined angle 48 within the range of 40° to 50° with respect to the actuation direction 24. The base body 12 comprises two hollow needle guiding sections 50 configured to guide the needles 30 and being arranged perpendicular to the longitudinal axis 46 of the inhaler 12 and ending in the capsule receptacle 36. Those needle guiding sections 50 and the needles 30 are arranged parallel to the actuation direction 24 and spaced apart from a middle plane 52 of the base body 12 in a distance 54. The needles 30 are arranged on opposing sides of the middle plane 52.

The circular cylindrical section 42 has a diameter 56 which is bigger than the length of the capsule receptacle 36. The size of the capsule receptacle 36 corresponds with the size of the capsule such that the capsule receptacle 36 is only slightly bigger than the capsule to ensure that the capsule is held in the capsule receptacle 36 when pushing the actuator buttons 22 and piercing the capsule with the needles 30. Preferably, the capsule receptacle 36 is configured to receive a capsule with a volume in the range of about 400 to about 500 mm³.

The base body 12 furthermore has two air inlets 58 in the area of the elliptical collar section 32 which are ending tangentially into the capsule chamber 36 and are arranged perpendicular to the middle plane 52 of the base body 12. The mouthpiece 14 is fastened to the base body 12 via a hinge. A lower part of the hinge is depicted in Figure 5 and having the reference numeral 59. The mouthpiece furthermore comprises two air inlets 60 fluidly connected with the air inlets 58 of the base body 12 via recesses 62 in the base body 12.

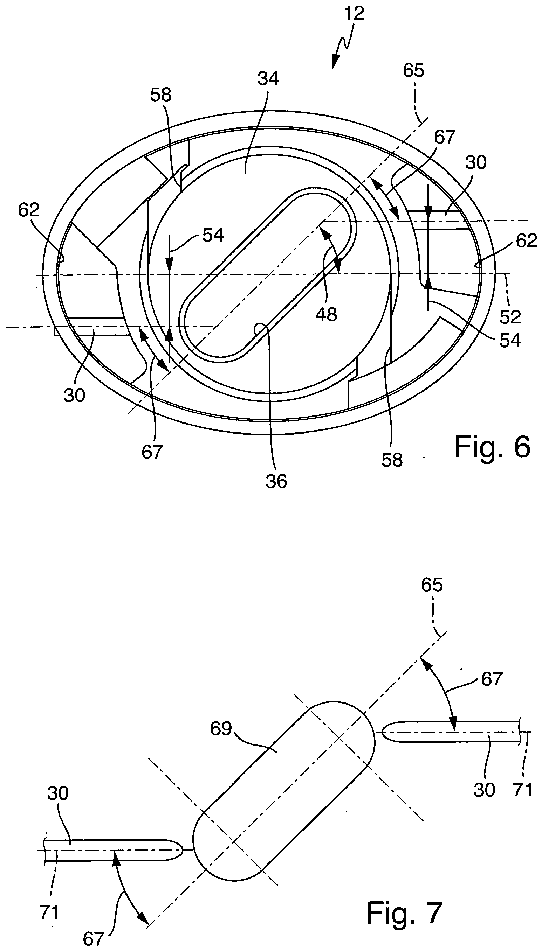

Figure 6 shows a top view of the base body 12 of Figure 5 when viewing in the direction of arrow 63 in Figure 5. The capsule receptacle 36 has a longitudinal axis 65 which is arranged in an inclined angle 48 within the range of about 40° to about 50° with respect to the actuation direction 24 or the middle plane 52 of the base body 12. The needles 30 are piercing a capsule which can be arranged in the capsule receptacle 36 in an angle 67 with respect to the longitudinal axis 65 of the capsule receptacle 36 or the capsule.

Such a capsule 69 is shown in Figure 7. The longitudinal axis 65 of the capsule 69 is arranged to a longitudinal axis 71 of the needles 30 in the angle 67.

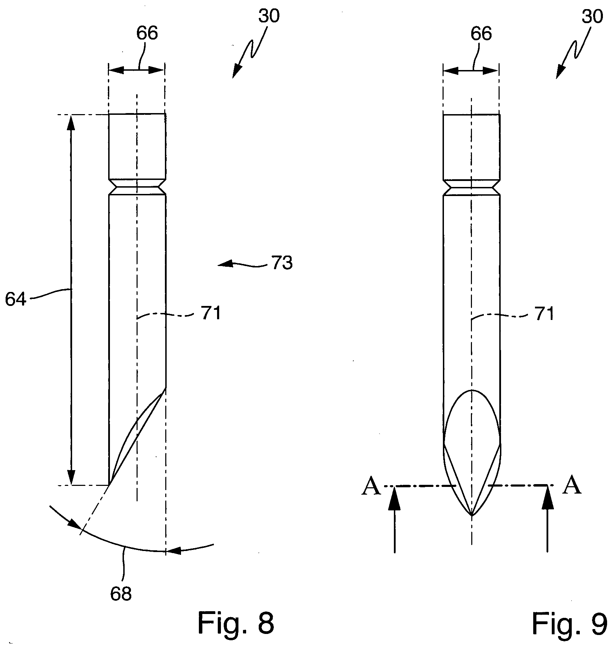

Figure 8 depicts a side view of one of the needles 30 of the dry powder inhaler 10. The needles 30 have a length 64, a needle diameter 66 and cutting tip angle 68. The needle diameter 66 is in the range of about 1,55 to about 1,85 mm wherein the cutting tip angle 68 is in the range of about 20° to 40°.

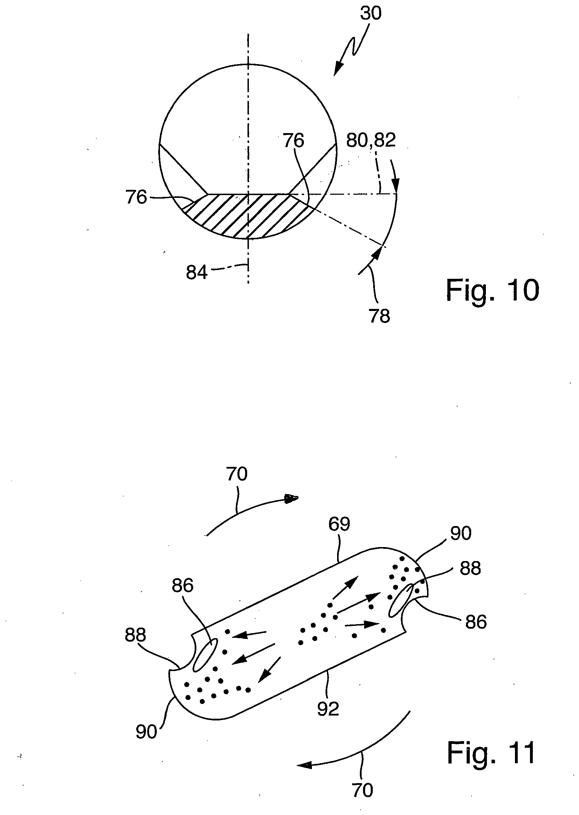

Figure 9 shows a top view of the needle 30 of Figure 8 when looking into the direction of arrow 73 in Figure 8. Figure 10 shows a partial cross-section A-A of the needle 30 of Figures 8 and 9 in a front view when looking into the direction of arrows 75 in Figure 9.

The needles 30 have lateral cutting edges 76 which are arranged in an angle 78 in the range of about 25 to 35° with respect to an axis 80 perpendicular to the longitudinal axis 71 of the needle 30 and lying in a plane 82 parallel to the longitudinal axis 71. The needles 30 are symmetrical to a middle plane 84.

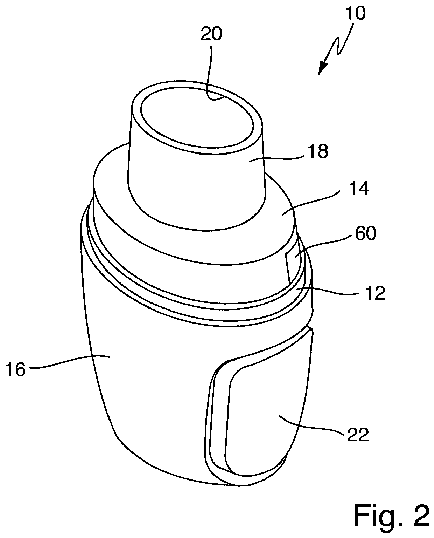

The dry powder inhaler 10 functions as follows:
In order to insert a dry powder capsule 69 into the capsule receptacle 36 of the base body 12, the mouthpiece 14 can be pivoted into an opening position via the hinge 59. After insertion of the capsule 69, the mouthpiece 14 is pivoted back into a closed position as depicted in Figures 1 and 2. In order to pierce the capsule 69, the actuator buttons 22 are pressed against the force of the helical spring 26 into the actuation direction 24.

The cutting tip angle 68, the lateral cutting edges 76 and the arrangement of the capsule receptacle 36 and the needles provide for an accurate cutting of a shell of the capsule 69 during piercing of the capsule 69 without detaching the cut-out portion from the capsule shell.

Figure 11 depicts a schematic view of a pierced capsule 69 during inhalation. Cut-out portions 86 can be bent inwards into the interior of the capsule 69 in a hinged or flap-like manner.

Because of the comparably large needle diameter 66, comparably big openings 88 can be pierced into the capsule 69 in a transition area of the hemispherical ends 90 and the tubular middle section of the capsule 69.

During inhalation of air through the central opening 20 of the mouthpiece 14, air is sucked in through the air inlets 58 and the air inlets 60. Because of the air flow in the capsule chamber 34, the capsule 69 is lifted from the capsule receptacle 36 into the cylindrical section 42 of the capsule chamber 34 and rotates into the direction of arrow 70.

Upon rotation of the capsule 69, dry powder is released or dispersed from the capsule 69 by centrifugal forces and mixed with ambient air which is sucked in through the air inlets 58, 60.

Because of the arrangement of the capsule receptacle 36 and the needles 30, the capsule 69 can be pierced in a position that allows powder to be efficiently dispersed during the rotational movement in an inhalation process while providing little or almost no obstruction to the powder flow.

To sum up, a dry powder inhaler 10 is provided which allows for a high dosage inhalation in the range of about 25 to about 200 mg.

## Claims

1. A dry powder inhaler (10) for a capsule (69) containing dry powder, the inhaler (10) comprising a base body (12) having a capsule receptacle (36), two actuator buttons (22) arranged on opposing sides of the base body (12) and two perforation needles (30), each needle (30) being fixedly connected to an actuator button (22) and movable relative to the base body (12) towards each other from a normal position to a perforation position along an actuation direction (24) to perforate a capsule (69) arranged in the capsule receptacle (36), **characterized in that** the capsule receptacle (36) is arranged in an inclined angle (48) within the range of about 40° to about 50° with respect to the actuation direction (24).

2. The dry powder inhaler (10) of claim 1, wherein the needles (30) are arranged parallel to the actuation direction (24) and spaced apart from a middle plane (52) of the base body (12).

3. The dry powder inhaler (10) of claim 1 or 2, wherein the capsule receptacle (36) is arranged perpendicular to a longitudinal axis (46) of the inhaler (10).

4. The dry powder inhaler (10) of at least one of the foregoing claims, wherein the base body (12) comprises two hollow needle guiding sections (50) configured to guide the needles (30), the needle guiding sections (50) being arranged perpendicular to a longitudinal axis (46) of the inhaler (10) and ending in the capsule receptacle (36).

5. The dry powder inhaler (10) of at least one of the foregoing claims, wherein the base body (12) is having a capsule chamber (34) for rotation of a capsule (69).

6. The dry powder inhaler (10) of claim 5, wherein the capsule receptacle (36) is arranged in the capsule chamber (34) and connected to the capsule chamber (34) .

7. The dry powder inhaler (10) of claims 5 or 6, wherein the capsule chamber (34) is having a circular cylindrical section (42) and a conical section (44), wherein the capsule receptacle (36) is arranged in the conical section (44).

8. The dry powder inhaler (10) of claim 7, wherein the circular cylindrical section (42) has diameter (56) which is bigger than a length of the capsule receptacle (36).

9. The dry powder inhaler (10) of one of claims 5 to 8, wherein the base body (12) is having at least two air inlets (58) connected to the capsule chamber (34) and ending tangentially in the capsule chamber (34).

10. The dry powder inhaler (10) of claim 9, wherein the dry powder inhaler (10) comprises a mouthpiece (14) arranged on top of the base body (12), wherein the mouthpiece (14) comprises at least one, advantageously two air inlets (60), fluidly connected with the air inlets (58) of the base body (12).

11. The dry powder inhaler (10) of at least one of the foregoing claims, wherein the capsule receptacle (36) is configured to receive a capsule (69) with a volume in the range of about 400 to about 500 mm³.

12. The dry powder inhaler (10) of claim 5, wherein the capsule chamber (34) has a volume of about 2500 to about 3000 mm³.

13. The dry powder inhaler (10) of at least one of the foregoing claims, wherein the needles (30) have a needle diameter (66) of about 1,55 to about 1,85 mm.

14. The dry powder inhaler (10) of at least one of the foregoing claims, wherein the needles (30) have a cutting tip with a cutting tip angle (68) with respect to a longitudinal axis (71) of the needle (30) in the range of about 20 to about 35°.

15. The dry powder inhaler (10) of claim 14, wherein the needles (30) have lateral cutting edges (76) which are arranged in an angle (78) in the range of about 25 to 35° with respect to an axis (80) perpendicular to the longitudinal axis (71) and lying in a plane (82) parallel to the longitudinal axis (71).

16. The dry powder inhaler (10) of at least one of the foregoing claims, wherein the needles (30) are symmetrical to a middle plane (84).

## Patentansprüche

1. Trockenpulverinhalator (10) für eine Kapsel (69), die Trockenpulver enthält, wobei der Inhalator (2) einen Basiskörper (12) mit einer Kapselaufnahme (36) umfasst, zwei an gegenüberliegenden Seiten des Basiskörpers (12) angeordnete Betätigungsknöpfe (22) und zwei Perforationsnadeln (30), wobei die Nadeln (30) jeweils fest mit einem Betätigungsknopf (22) verbunden und in Bezug auf den Basiskörper (12) von einer Normalstellung in eine Perforationsstellung entlang einer Betätigungsrichtung (24) zueinander beweglich sind, um eine in der Kapselaufnahme (36) befindliche Kapsel (69) zu perforieren, **dadurch gekennzeichnet, dass** die Kapselaufnahme (36) in Bezug auf die Betätigungsrichtung (24) in einem schrägen Winkel (48) im Bereich zwischen etwa 40 und etwa 50° angeordnet ist.

2. Trockenpulverinhalator (10) nach Anspruch 1, wobei die Nadeln (30) parallel zu der Betätigungsrichtung (24) angeordnet und von einer Mittelebene (52) des Basiskörpers (12) beabstandet sind.

3. Trockenpulverinhalator (10) nach Anspruch 1 oder 2, wobei die Kapselaufnahme (36) senkrecht zu einer Längsachse (46) des Inhalators (10) angeordnet ist.

4. Trockenpulverinhalator (10) nach mindestens einem der vorstehenden Ansprüche, wobei der Basiskörper (12) zwei hohle Nadelführungsabschnitte (50) umfasst, die zur Führung der Nadeln (30) dienen, wobei die Nadelführungsabschnitte (50) senkrecht zu einer Längsachse (46) des Inhalators (10) angeordnet sind und in der Kapselaufnahme (36) enden.

5. Trockenpulverinhalator (10) nach mindestens einem der vorstehenden Ansprüche, wobei der Basiskörper (12) eine Kapselkammer (34) umfasst, die zur Drehung einer Kapsel (69) dient.

6. Trockenpulverinhalator (10) nach Anspruch 5, wobei die Kapselaufnahme (36) in der Kapselkammer (34) angeordnet und mit der Kapselkammer (34) verbunden ist.

7. Trockenpulverinhalator (10) nach Anspruch 5 oder 6, wobei die Kapselkammer (34) einen kreiszylindrischen Abschnitt (42) und einen kegelförmigen Abschnitt (44) aufweist und die Kapselaufnahme (36) in dem kegelförmigen Abschnitt (44) angeordnet ist.

8. Trockenpulverinhalator (10) nach Anspruch 7, wobei der kreiszylindrische Abschnitt (42) einen Durchmesser (56) aufweist, der größer ist als eine Länge der Kapselaufnahme (36).

9. Trockenpulverinhalator (10) nach einem der Ansprüche 5 bis 8, wobei der Basiskörper (12) mindestens zwei Lufteinlässe (58) aufweist, die mit der Kapselkammer (34) verbunden sind und tangential in der Kapselkammer (34) enden.

10. Trockenpulverinhalator (10) nach Anspruch 9, wobei der Trockenpulverinhalator (10) ein Mundstück (14) umfasst, das am oberen Ende des Basiskörpers (12) angeordnet ist und mindestens einen, bevorzugt zwei Lufteinlässe (60) aufweist, die mit den Lufteinlässen (58) des Basiskörpers (12) fluidisch verbunden sind.

11. Trockenpulverinhalator (10) nach mindestens einem der vorstehenden Ansprüche, wobei die Kapselaufnahme (36) so ausgelegt ist, dass sie eine Kapsel (69) mit einem Volumen im Bereich zwischen etwa 400 und etwa 500 mm³ aufnehmen kann.

12. Trockenpulverinhalator (10) nach Anspruch 5, wobei die Kapselkammer (34) ein Volumen zwischen etwa 2500 und etwa 3000 mm³ hat.

13. Trockenpulverinhalator (10) nach mindestens einem der vorstehenden Ansprüche, wobei die Nadeln (30) einen Nadeldurchmesser (66) zwischen etwa 1,55 und etwa 1,85 mm aufweisen.

14. Trockenpulverinhalator (10) nach mindestens einem der vorstehenden Ansprüche, wobei die Nadeln (30) eine Schneidspitze umfassen, die in Bezug auf eine Längsachse (71) der Nadel (30) einen Schneidspitzenwinkel (68) im Bereich zwischen etwa 20 und etwa 35° aufweist.

15. Trockenpulverinhalator (10) nach Anspruch 14, wobei die Nadeln (30) seitliche Schneidkanten (76) aufweisen, die in Bezug auf eine Achse (80) senkrecht zu der Längsachse (71) in einem Winkel (78) im Bereich zwischen etwa 25 und 35° angeordnet sind und in einer Ebene (82) liegen, die parallel zu der Längsachse (71) verläuft.

16. Trockenpulverinhalator (10) nach mindestens einem der vorstehenden Ansprüche, wobei die Nadeln (30) symmetrisch zu einer Mittelebene (84) angeordnet sind.

## Revendications

1. Inhalateur de poudre sèche (10) pour une capsule (69) contenant de la poudre sèche, l'inhalateur (10) comprenant un corps de base (12) comportant un réceptacle de capsule (36), deux boutons d'actionnement (22) agencés sur des côtés opposés du corps de base (12) et deux aiguilles de perforation (30), chaque aiguille (30) étant reliée de manière fixe à un bouton d'actionnement (22) et pouvant être déplacée l'une vers l'autre par rapport au corps de base (12) à partir d'une position normale vers une position de perforation le long d'un direction d'actionnement (24) pour perforer une capsule (69) agencée dans le réceptacle de capsule (36), **caractérisé en ce que** le réceptacle de capsule (36) est agencé selon un angle incliné (48) dans la plage d'environ 40° à environ 50° par rapport à la direction d'actionnement (24) .

2. Inhalateur de poudre sèche (10) selon la revendication 1, dans lequel les aiguilles (30) sont disposées parallèlement à la direction d'actionnement (24) et espacées par rapport à un plan médian (52) du corps de base (12).

3. Inhalateur de poudre sèche (10) selon la revendication 1 ou 2, dans lequel le réceptacle de capsule (36) est agencé perpendiculairement à un axe longitudinal (46) de l'inhalateur (10).

4. Inhalateur de poudre sèche (10) selon au moins l'une des revendications précédentes, dans lequel le corps de base (12) comprend deux sections de guidage d'aiguille creuse (50) configurées pour guider les aiguilles (30), les sections de guidage d'aiguille (50) étant agencées perpendiculairement à un axe longitudinal (46) de l'inhalateur (10) et se terminant dans le réceptacle de capsule (36).

5. Inhalateur de poudre sèche (10) selon au moins l'une des revendications précédentes, dans lequel le corps de base (12) présente une chambre de capsule (34) pour la rotation d'une capsule (69).

6. Inhalateur de poudre sèche (10) selon la revendication 5, dans lequel le réceptacle de capsule (36) est disposé dans la chambre de capsule (34) et relié à la chambre à capsule (34).

7. Inhalateur de poudre sèche (10) selon la revendication 5 ou 6, dans lequel la chambre à capsule (34) présente une section cylindrique circulaire (42) et une section conique (44), dans lequel le réceptacle de capsule (36) est agencé dans la section conique (44).

8. Inhalateur de poudre sèche (10) selon la revendication 7, dans lequel la section cylindrique circulaire (42) a un diamètre (56) qui est supérieur à une longueur du réceptacle de capsule (36).

9. Inhalateur de poudre sèche (10) selon l'une des revendications 5 à 8, dans lequel le corps de base (12) présente au moins deux entrées d'air (58) reliées à la chambre de capsule (34) et se terminant de manière tangentielle dans la chambre de capsule (34).

10. Inhalateur de poudre sèche (10) selon la revendication 9, dans lequel l'inhalateur de poudre sèche (10) comprend un embout buccal (14) agencé sur le dessus du corps de base (12), dans lequel l'embout buccal (14) comprenant au moins une, avantageusement deux entrées d'air (60), reliées de manière fluide aux entrées d'air (58) du corps de base (12).

11. Inhalateur de poudre sèche (10) selon au moins l'une des revendications précédentes, dans lequel le réceptacle de capsule (36) est configuré pour recevoir une capsule (69) ayant un volume compris entre environ 400 et environ 500 mm³.

12. Inhalateur de poudre sèche (10) selon la revendication 5, dans lequel la chambre de capsule (34) a un volume d'environ 2500 à environ 3000 mm³.

13. Inhalateur de poudre sèche (10) selon au moins l'une des revendications précédentes, dans lequel les aiguilles (30) ont un diamètre d'aiguille (66) d'environ 1,55 à environ 1,85 mm.

14. Inhalateur de poudre sèche (10) selon au moins l'une des revendications précédentes, dans lequel les aiguilles (30) ont une pointe coupante avec un angle de pointe coupante (68) par rapport à un axe longitudinal (71) de l'aiguille (30) dans la plage d'environ 20 à environ 35°.

15. Inhalateur de poudre sèche (10) selon la revendication 14, dans lequel les aiguilles (30) ont des bords coupants latéraux (76) qui sont agencés selon un angle (78) dans la plage d'environ 25 à 35° par rapport à un axe (80) perpendiculaire à l'axe longitudinal (71) et situé dans un plan (82) parallèle à l'axe longitudinal (71).

16. Inhalateur de poudre sèche (10) selon au moins l'une des revendications précédentes, dans lequel les aiguilles (30) sont symétriques par rapport à un plan médian (84).
